# EUROPEAN PATENT APPLICATION

(11) **EP 4 555 978 A1**
(43) Date of publication of application: **21.05.2025**
(21) Application number: 23839657.6
(22) Date of filing: 12.07.2023
(51) Int. Cl.: A61F 2/90, A61B 17/22, A61F 2/01, A61F 2/844, A61M 25/00, A61M 25/09

(54) **DISTAL STABILIZER**

(30) Priority: 14.07.2022 JP 2022113422
(71) Applicant: Bolt Medical Inc., Tokyo 103-0023 (JP)
(72) Inventor: INUZUKA, Naoki, Tokyo 103-0023 (JP)
(74) Representative: Appleyard Lees IP LLP
(86) International application number: PCT/JP2023/025753
(87) International publication number: WO 2024/014485

(57) **Abstract**

Provided is a distal stabilizer capable of suppressing a positional misalignment of a cylindrical part locked in a biological lumen. This distal stabilizer 1, used for catheter delivery in a biological lumen, comprises: a linear delivery member; and a cylindrical part 2 which extends from a distal end of the linear delivery member and locked to the inner wall of the biological lumen by means of a mesh pattern structure, wherein the cylindrical part 2 has, on the proximal side in the range of the effective length thereof, an easily deformable section 14 which more easily deforms than the distal side of the cylindrical part.

## Description

### TECHNICAL FIELD

The present invention relates to a distal stabilizer for use in a catheter delivery in a biological lumen.

### BACKGROUND ART

In a biological lumen such as an artery of a patient, a treatment using a treatment device or a treatment using the catheter itself as a treatment device is performed by delivering a treatment device to a target position using a passage inside a catheter whose distal end is guided to the vicinity of the target position. For example, Patent Document 1 discloses an anchoring device in which a cylindrical portion (locking stent) is joined to the distal end of a delivery wire. When the cylindrical portion is released from a microcatheter and expanded, since the cylindrical portion is locked (anchored) to the inner wall in the biological lumen, the operation of delivering the treatment catheter that is slidably fitted over the microcatheter to the vicinity of the target position can be easily performed.

### Citation List

### Patent Document

Patent Document 1: US Patent No. 968221

### DISCLOSURE OF THE INVENTION

### Problems to be Solved by the Invention

The treatment catheter that is slidably fitted over the microcatheter has a large outer diameter and high rigidity. Therefore, the treatment catheter repeatedly applies a force to pull the microcatheter or the delivery wire having low rigidity to the proximal side during the advancement. In particular, in a case in which a blood vessel, which is a biological lumen, includes a highly bent tortuous blood vessel, the treatment catheter is easily caught on a bent portion or a bifurcated portion, and when a force is applied to make the treatment catheter to pass through the bent portion or the bifurcated portion, a force for pulling the microcatheter or the delivery wire to the proximal side is applied repeatedly.

The application of the force to pull the delivery wire to the proximal side may cause a displacement of the cylindrical portion locked to the inner wall in the biological lumen. When the cylindrical portion is displaced, it is necessary to store the cylindrical portion in the microcatheter, release the cylindrical portion from the distal side of the microcatheter in the vicinity of the target position, and lock the cylindrical portion in the biological lumen. Therefore, it takes time and effort to deliver the treatment catheter to the vicinity of the target location.

An object of the present invention is to provide a distal stabilizer capable of suppressing displacement of a cylindrical portion locked in a biological lumen.

### Means for Solving the Problems

The present invention is directed to a distal stabilizer for use in catheter delivery in a biological lumen, the distal stabilizer including: a linear delivery member; and a cylindrical portion that extends from a distal end of the linear delivery member and is locked to an inner wall of the biological lumen by a mesh pattern structure, in which the cylindrical portion has a range of an effective length, and includes an easily deformable portion on a proximal side of the range of the effective length, such that the proximal side is more deformable than a distal side of the effective length.

An opening area of each cell constituting the mesh pattern structure of the easily deformable portion may be larger than an opening area of each cell constituting the mesh pattern structure of the distal side of the cylindrical portion.

An opening area of each cell constituting the mesh pattern structure of the easily deformable portion may be larger than a reference opening area.

A ratio of a length of the easily deformable portion in an axial direction relative to the range of the effective length of the cylindrical portion may be 1:0.5 to 1:0.2.

### Effects of the Invention

According to the present invention, it is possible to provide a distal stabilizer capable of suppressing displacement of a cylindrical portion locked in a biological lumen.

### BRIEF DESCRIPTION OF THE DRAWINGS

FIG. 1 shows a delivery system 10 including a distal stabilizer 1 according to an embodiment;
FIG. 2 is a perspective view showing a state in which a main body portion 11 of a locking stent 2 is virtually expanded in a plane;
FIG. 3 is a schematic diagram illustrating an example of a procedure performed by the delivery system 10 including the distal stabilizer 1;
FIG. 4 is a schematic diagram illustrating an example of a procedure performed by the delivery system 10 including the distal stabilizer 1;
FIG. 5 is a schematic diagram illustrating an example of a procedure performed by the delivery system 10 including the distal stabilizer 1;
FIG. 6 is a schematic diagram illustrating an example of a procedure performed by the delivery system 10 including the distal stabilizer 1;
FIG. 7 is a schematic diagram illustrating an example of a procedure performed by the delivery system 10 including the distal stabilizer 1;
FIG. 8 is a schematic diagram illustrating an example of a procedure performed by the delivery system 10 including the distal stabilizer 1;
FIG. 9 is a schematic diagram illustrating an example of a procedure performed by the delivery system 10 including the distal stabilizer 1;
FIG. 10 is a schematic diagram illustrating an example of a procedure performed by the delivery system 10 including the distal stabilizer 1;
FIG. 11 is a schematic diagram illustrating an example of a procedure performed by the delivery system 10 including the distal stabilizer 1;
FIG. 12 is an enlarged view showing the shape of the locking stent 2 locked in the biological lumen V;
FIG. 13 is an enlarged view showing a shape of a locking stent 2A of a comparative example when a force for pulling the first catheter 5 and the delivery wire 3 toward the proximal side D1 is applied; and
FIG. 14 is an enlarged view showing the shape of the locking stent 2 when a force is applied to pull the first catheter 5 and the delivery wire 3 toward the proximal side D1.

### PREFERRED MODE FOR CARRYING OUT THE INVENTION

Hereinafter, embodiments of the distal stabilizer according to the present invention will be described. It should be noted that all of the drawings attached to the present specification are schematic diagrams, and the shape, the scale, the vertical and horizontal dimensional ratio, and the like of each part are changed or exaggerated from actual ones in consideration of ease of understanding and the like. For example, the longitudinal direction of a catheter or the like is shown to be shorter, and the radial direction is shown to be thicker. In this specification, etc., terms specifying a shape, a geometric condition, and a degree thereof, for example, a term such as "direction", includes a range generally regarded as the direction in addition to the strict meaning of the term. In addition, in the present specification, the long axis direction in a state where the distal stabilizer 1 extends linearly is also referred to as an "axial direction LD" or simply as an "axial direction". In the axial direction LD, the proximal side close to the practitioner is referred to as "D1", and the distal side away from the practitioner is referred to as "D2".

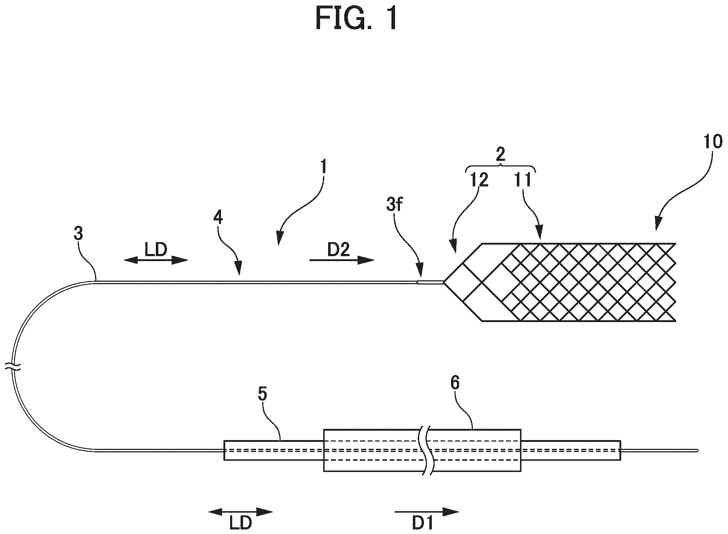

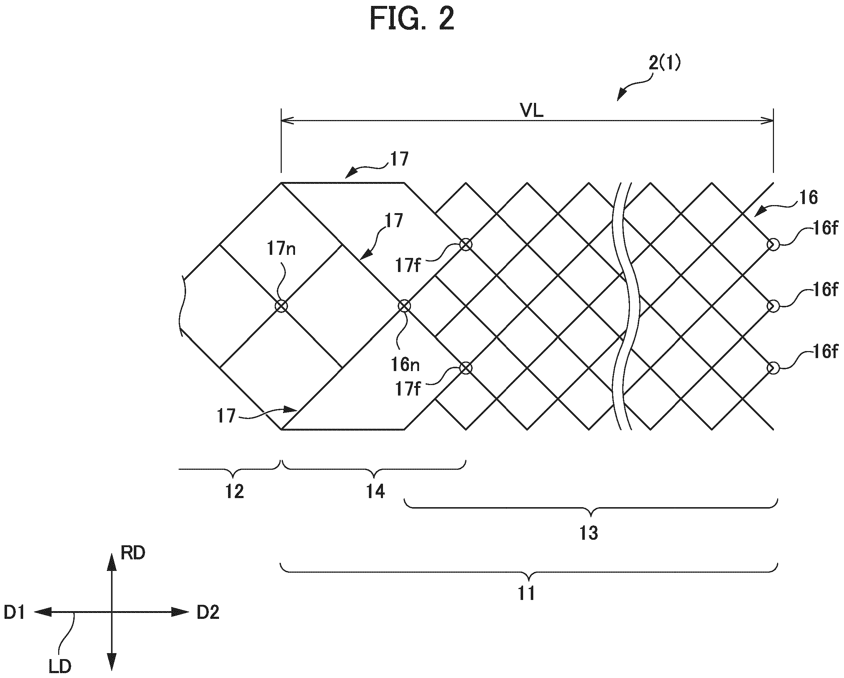

FIG. 1 shows a delivery system 10 including a distal stabilizer 1 according to an embodiment. FIG. 2 is a perspective view showing a state in which a main body portion 11 of a locking stent 2 is virtually expanded in a plane.

The delivery system 10 shown in FIG. 1 is a system used to deliver a treatment device (described below) in a biological lumen. The blood vessels of the biological lumen in which the delivery system 10 is used are not particularly limited, but typically include tortuous large cerebral blood vessels and the like.

As shown in FIG. 1, the delivery system 10 includes the distal stabilizer 1 and a plurality of catheters, including a first catheter 5 and a second catheter 6. The distal stabilizer 1 is a device used for catheter delivery in a biological lumen. The distal stabilizer 1 includes the locking stent (cylindrical portion) 2 and a delivery wire (linear delivery member) 3. In the present disclosure, each "cell" described later refers to a portion surrounded by portions made of a wire-shaped material forming a mesh pattern structure. Each "strut" refers to an elongated rod-shaped or band-shaped portion made of the wire-shaped material.

### (Locking Stent)

The locking stent 2 is an anchoring device that extends from a distal end 3f of the delivery wire 3 and is lockable to the inner wall of the biological lumen by a self-expanding force. The locking stent 2 is housed in the first catheter 5 in a state reduced in diameter, and is locked to the inner wall of the biological lumen by being released from the first catheter 5 and deployed in the biological lumen. The biological lumen is not particularly limited, and may be a blood vessel (an artery or a vein) of the brain, a coronary artery, an upper or lower limb, or the like, an organ, or the like. The locking stent 2 includes a main body portion 11 and an antenna portion 12.

The main body portion 11 is a portion configured to have a cylindrical shape in a state expanded in diameter, and has a mesh pattern structure described later. As shown in FIG. 2, the range of the main body portion 11 in the axial direction LD corresponds to the range of the effective length VL of the locking stent 2. The range of the effective length VL refers to a range between distal end portions 16f of cells 16 (described later) located on the most distal side D2 of the mesh pattern structure and a proximal end portion 17n of the cell 17 (described later) located on the most proximal side D1. In the locking stent 2 of the present embodiment, there are a plurality of cells 16 located on the most distal side D2 of the mesh pattern structure and a plurality of cells 17 located on the most proximal side D1. However, the number of cells located on the most distal side and the most proximal side may be one, respectively. The locking stent 2 can support the inner wall of the biological lumen by increasing the diameter in the main body portion 11 (the range of the effective length VL). In the drawings of the present embodiment, the mesh pattern structure of the main body portion 11 is simplified.

As shown in FIG. 1, the antenna portion 12 is a portion at which a portion on the proximal side of the main body portion 11 converges to the distal end 3f of the delivery wire 3. The antenna portion 12 has a mesh pattern structure and is designed so as not to support the inner wall of the biological lumen when expanded at the inner diameter of the blood vessel at the target position. The locking stent 2 includes, at its distal and proximal ends, markers (not shown), each made of X-ray opaque material. Each of the markers of the locking stent 2 is a mark for confirming the position of the distal end and the proximal end of the locking stent 2 in the X-ray transmission image.

The locking stent 2 can be made, for example, by performing laser processing on a tube made of a biocompatible material, particularly preferably a superelastic alloy. In a case of producing the locking stent 2 from a superelastic alloy tube, in order to reduce the cost, it is preferable to produce the locking stent 2 by performing laser processing on a tube of about 2 to 3 mm, then expanding the tube to a desired diameter, and subjecting the tube to shape memory treatment. The locking stent 2 is not limited to laser processing, and may be produced by cutting or the like, or may be produced by braiding a wire-shaped metal wire into a tubular shape. In a case in which the material or thickness of the strut is changed depending on the region of the locking stent 2 in the axial direction, if the material is metallic, two mesh portions to be described later may be joined by welding or the like.

The locking stent 2 is preferably made of a material having high rigidity and high biocompatibility. Examples of such a material include titanium, nickel, stainless steel, platinum, gold, silver, copper, iron, chromium, cobalt, aluminum, molybdenum, manganese, tantalum, tungsten, niobium, magnesium, calcium, and alloys containing these. Further, as such a material, for example, a synthetic resin material of a polyolefin such as polyethylene (PE) or polypropylene (PP), polyamide, polyvinyl chloride, polyphenylene sulfide, polycarbonate, polyether, polymethyl methacrylate or the like may be used. Further, as such a material, for example, a biodegradable resin (biodegradable polymer) such as polylactic acid (PLA), polyhydroxybutyrate (PHB), polyglycolic acid (PGA), or poly ε-caprolactone may be used.

Among these, titanium, nickel, stainless steel, platinum, gold, silver, copper, magnesium, and alloys containing these are preferable. Examples of the alloy include a Ni-Ti alloy, a Cu-Mn alloy, a Cu-Cd alloy, a Co-Cr alloy, a Cu-Al-Mn alloy, an Au-Cd-Ag alloy, and a Ti-Al-V alloy. Further, examples of the alloy include an alloy of magnesium and Zr, Y, Ti, Ta, Nd, Nb, Zn, Ca, Al, Li, Mn, or the like. Among these alloys, a Ni-Ti alloy is preferable.

As shown in FIG. 2, in the main body 11, a plurality of cells (cells 16 and 17 to be described later) defined by a plurality of struts arranged in a frame shape are spread in the radial direction (circumferential direction) RD. A part of the plurality of cells spread in the radial direction RD is continuously arranged along the axial direction LD. That is, the main body portion 11 has a mesh pattern structure in which a plurality of cells are spread in the radial direction RD and a part of the cells is continuously arranged along the axial direction LD. The circumferential direction refers to an outer circumferential direction of the main body portion 11. The radial direction RD (a direction orthogonal to the axial direction LD) shown in FIG. 2 corresponds to the circumferential direction in the main body portion 11 virtually expanded in a plane.

In the main body portion 11, the mesh pattern structure includes a first mesh portion 13 and a second mesh portion (an easily deformable portion) 14. The first mesh portion 13 and the second mesh portion 14 are regions having different ease of deformation in the main body portion 11. In the main body portion 11, the second mesh portion 14, which is more easily deformed than the first mesh portion 13, is adjacent to the first mesh portion 13 in the axial direction. As shown in FIG. 2, in the mesh pattern structure of the present embodiment, the first mesh portion 13 and the second mesh portion 14 partially overlap each other in the circumferential direction, but may be a configuration not overlapping. In addition, in the configuration in which the first mesh portion 13 and the second mesh portion 14 partially overlap each other in the circumferential direction, an intermediate position between the first mesh portion 13 and the second mesh portion 14 may be defined as a boundary between the first mesh portion 13 and the second mesh portion 14 in the axial direction LD. As shown in FIG. 2, in the mesh pattern structure of the present embodiment, the boundary between the first mesh portion 13 and the second mesh portion 14 has a zigzag pattern shape along the circumferential direction. However, for example, even in a mesh pattern structure in which the first mesh portion 13 and the second mesh portion 14 are arranged in a spiral shape, its boundary can be specified by the same method.

### (First Mesh Portion)

The first mesh portion 13 is a portion of the main body portion 11 located on the distal side D2 in the axial direction LD. The first mesh portion 13 has a self-expanding force, and expands by being released from the catheter to support the inner wall of the blood vessel. Even when the delivery wire 3 is pulled toward the proximal side D1, the first mesh portion 13 is in a state of supporting the inner wall of the blood vessel. Therefore, the first mesh portion 13 expands to support the inner wall of the blood vessel, such that the blood flow is secured and the locking stent 2 can be anchored to the inner wall of the blood vessel. As shown in FIG. 2, the first mesh portion 13 includes the plurality of cells 16. In FIG. 2, the first mesh portion 13 is provided between a proximal end portion 16n of the cell 16 located on the most proximal side D1 in the axial direction LD and the distal end portions 16f of the cells 16 located on the most distal side D2. In the present embodiment, each of the cells 16 constituting the first mesh portion 13 has substantially the same shape, and therefore has substantially the same opening area. The opening area of each of the cells 16 is, for example, about 1 to 10 mm². As the opening area of each of the cells 16, for example, a value obtained by arithmetic mean from the opening areas of all of the cells 16 constituting the first mesh portion 13 can be used.

The cell pattern of the cells 16 constituting the first mesh portion 13 is not particularly limited, but may be all open cells or all closed cells. As a cell pattern of the cell 16, closed cells and open cells may coexist. It should be noted that, in the present disclosure, the definitions of an open cell and a closed cell are not uniquely defined. For example, in a closed cell including a free end, a half region surrounded by two sides (struts) forming the free end may be defined as an open cell, and the other half region may be defined as a closed cell, or alternatively, a closed cell including a free end may be defined as an open cell, and a closed cell not including a free end may be defined as a closed cell.

### (Second Mesh Portion)

As shown in FIG. 2, the second mesh portion 14 is a portion located more to the proximal side D1 of the main body portion 11 than the first mesh portion 13. The second mesh portion 14 has a self-expanding force and expands by being released from the catheter to support the inner wall of the blood vessel. However, when the delivery wire 3 is pulled and drawn toward the proximal side D1, the second mesh portion 14 no longer supports the inner wall of the blood vessel. As shown in FIG. 2, the second mesh portion 14 includes the plurality of cells 17. In FIG. 2, the second mesh portion 14 is provided between the proximal end portion 17n of the cell 17 located on the most proximal side D1 in the axial direction LD and distal end portions 17f of the cells 17 located on the most distal side D2. The second mesh portion 14 of the present embodiment includes three cells 17, but the number of cells may be four or more or less than three. The shapes of the cells 17 constituting the second mesh portion 14 may be different from one another or may be the same. The cell pattern of the cells 17 constituting the second mesh portion 14 is not particularly limited, but may be all open cells or all closed cells. As a pattern of the cell 17, closed cells and open cells may coexist.

The second mesh portion 14 is provided as an easily deformable portion that is more easily deformed than the first mesh portion 13. Specifically, the second mesh portion 14 extends more easily in the axial direction than the first mesh portion 13 and has a lower bending rigidity in the axial direction. The elongation ratio of each of the first mesh portion 13 and the second mesh portion 14 can be calculated as a ratio to the original sample length, for example, by forming each portion to have a predetermined sample length, loading the portions into a small diameter tube, and measuring the length in the axial direction. The diameter of the small diameter tube used in the test may be, for example, the smallest diameter of the blood vessel in which the distal stabilizer 1 is used. The elongation ratio of the second mesh portion 14 is preferably, for example, about 150 to 300% of the elongation ratio of the first mesh portion 13.

The bending rigidity in the axial direction of each of the first mesh portion 13 and the second mesh portion 14 can be measured by forming each portion to have a predetermined sample length and performing, for example, a three-point bending test, a four-point bending test, or the like. The bending rigidity of the second mesh portion 14 is preferably about 30% to 70% of the bending rigidity of the first mesh portion 13. For example, when the bending rigidity of the first mesh portion 13 is 10 to 20 Nmm², the bending rigidity of the second mesh portion 14 is preferably about 3 to 14 Nmm². The second mesh portion 14 can be formed by, for example, entirely forming the first mesh portion 13 as the main body portion 11 and removing a portion of the struts in the region on the proximal side D1.

The second mesh portion 14 is provided so that the opening area of each of the cells 17 constituting the second mesh portion 14 is larger than the opening area of each of the cells 16 constituting the first mesh portion 13 such that the second mesh portion 14 easily extends in the axial direction more than the first mesh portion 13 and the bending rigidity in the axial direction is lower than the first mesh portion 13. Although depending on the opening area of each of the cells 16 of the first mesh portion 13, the opening area of each of the cells 17 is, for example, about 6 to 60 mm². As the opening area of each of the cells 17, for example, a value obtained by arithmetic mean from the opening areas of all of the cells 17 constituting the second mesh portion 14 can be used.

As a configuration in which the second mesh portion 14 easily extends in the axial direction more than the first mesh portion 13 and the bending rigidity in the axial direction is lower than the first mesh portion 13, the opening area of each of the cells 17 of the second mesh portion 14 may be larger than the reference opening area. The reference opening area is an opening area serving as a reference when determining the magnitude of the bending rigidity in the axial direction in the mesh pattern structure. The reference opening area is selected according to the degree of flexibility imparted to the second mesh portion 14. By setting the opening area of each of the cells 17 of the second mesh portion 14 based on the reference opening area, it is possible to more accurately and quantitatively set the ease of deformation of the second mesh portion 14 with respect to the first mesh portion 13.

In the locking stent 2, the ratio of the length of the second mesh portion 14 (the easily deformable portion) in the axial direction to the main body portion 11 (the range of the effective length VL) is preferably 1:0.5 to 1:0.2 (the main body portion: the second mesh portion). When the ratio of the length of the second mesh portion 14 in the axial direction to the main body portion 11 is less than 1:0.5 (lower limit value), it becomes difficult for the main body portion 11 to secure a locking force necessary for locking the locking stent 2 to the inner wall of the biological lumen. In addition, when the ratio of the length of the second mesh portion 14 in the axial direction to the main body portion 11 exceeds 1:0.2 (the upper limit value), the characteristics of the second mesh portion 14 (elongation and bending in the axial direction described later) when the delivery wire 3 is pulled toward the proximal side are impaired. Therefore, by setting the ratio of the length of the second mesh portion 14 in the axial direction to the main body portion 11 to 1:0.5 to 1:0.2, it is possible to sufficiently exhibit the characteristics of the second mesh portion 14, while securing the locking force of the first mesh portion 13.

Returning to FIG. 1, the delivery wire 3 is a member used when the locking stent 2 is advanced or retracted in the biological lumen. The delivery wire 3 is delivered toward the distal side D2 when the locking stent 2 is advanced within the biological lumen V, and is drawn toward the proximal side D1 when the locking stent 2 is retracted within the biological lumen V. The delivery wire 3 is made of, for example, a metal material having a high elastic modulus such as stainless steel. In addition, the diameter of the delivery wire 3 is not particularly limited as long as it has physical properties sufficient to perform an advancing or retracting operation in the biological lumen V and is adapted to the first catheter 5, and may be, for example, 0.005 to 0.018 inches. The linear delivery member is not limited to a metal material such as a delivery wire, and may be made of, for example, a resin or a composite material of a metal and a resin.

As shown in FIG. 1, the first catheter 5 is slidably fitted over the delivery wire 3. The first catheter 5 is, for example, a microcatheter. The second catheter 6 (described later) is slidably fitted over the first catheter 5. The diameter of the first catheter 5 is set according to a target position TP, and the inner diameter and the degree of bending of a biological lumen V of the path to the target position TP, and is not particularly limited, but the inner diameter is preferably 0.017 inches or less, and more preferably 0.0165 inches or less. As the catheter, one or more other catheters (not shown) slidably fitted over the second catheter 6 may be used as necessary. In general, by using a large number of catheters, a catheter having a large inner diameter can be ultimately inserted into the biological lumen V and advanced.

Among the plurality of catheters including the second catheter 6, a catheter having an inner diameter larger than that of the first catheter 5 is referred to as a treatment catheter. The treatment catheter is a catheter having an inner diameter sufficient to place the treatment device thereinside in an inserted manner or having an inner diameter sufficient to use itself as a treatment device. The treatment catheter may also be referred to as a guiding catheter in applications that place the treatment device thereinside in an inserted manner. Examples of the treatment device include a thrombus aspiration device, a flow diverter, an aneurysm embolization device, a thrombectomy device (such as a stent retriever), a stent for treating aneurysm, a stent for treating intracranial arterial stenosis, a balloon catheter, a shunt, and a liquid embolic substance release means (such as a catheter having a lumen through which a liquid embolic substance passes). The treatment catheter may itself be used as a treatment device. In such applications, the treatment catheter may also be referred to as a thrombus aspiration catheter. In the embodiment described below, a case where the second catheter 6 is a treatment catheter will be described as an example.

Next, some procedures in the operation of delivering the treatment device to the target location TP using the delivery system 10 of the embodiment will be described. Although various operations other than those described below are performed as the procedure, a description thereof is omitted here. In the present embodiment, the biological lumen V is a blood vessel. In particular, the distal stabilizer 1 and the delivery system 10 of the present embodiment are suitably used when a highly bent tortuous blood vessel is included in a blood vessel. Further, the distal stabilizer 1 and the delivery system 10 of the present embodiment are preferably used when the target position TP is located in a region having a blood vessel inner diameter of 7 mm or less, specifically, less than 2.5 mm (preferably, 2.0 mm or less or 1.5 mm or less). Specifically, the target position TP may be a region after M2 (M2, M3, M4, etc.) of the middle cerebral artery (MCA), A1 and A2 regions of the anterior cerebral artery (ACA), a region after P1 (P1, P2, etc.) of the posterior cerebral artery (PCA), the internal carotid artery (ICA), etc. However, the target position TP is not limited thereto, and may be located in a wide range of a blood vessel inner diameter of 0.5 to 10 mm.

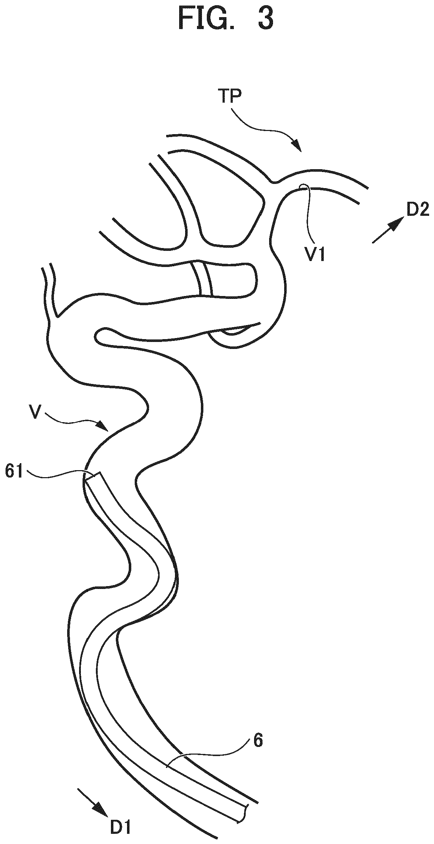

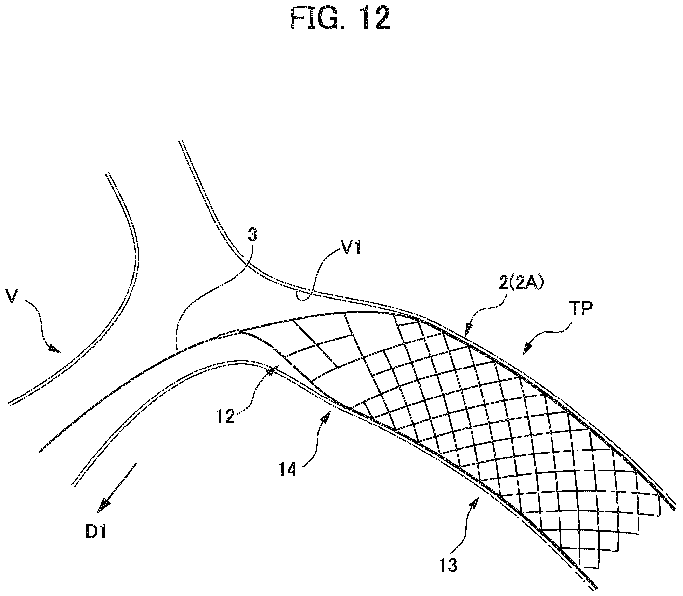

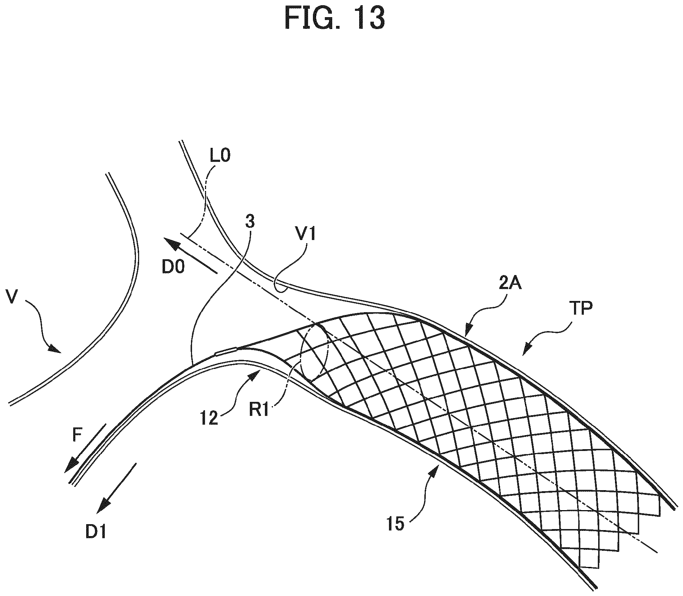

FIGS. 3 to 11 are schematic diagrams each illustrating an example of a procedure performed by the delivery system 10 including the distal stabilizer 1. FIG. 12 is an enlarged view showing the shape of the locking stent 2 (2A) locked in the biological lumen V. Since the shape of the locking stent in a state of being locked in the biological lumen V and not being pulled toward the proximal side D1 is substantially the same in both the locking stent of the embodiment and the locking stent of the comparative example, the locking stent 2 of the embodiment is representatively illustrated in FIG. 12. As shown in FIG. 13, the mesh pattern structure of the locking stent 2A of the comparative example includes cells having the same shape as a whole. In the locking stent 2A of the comparative example shown in FIG. 13, the mesh pattern structure is the same as that of the first mesh portion 13, and will be described as a "mesh portion 15".

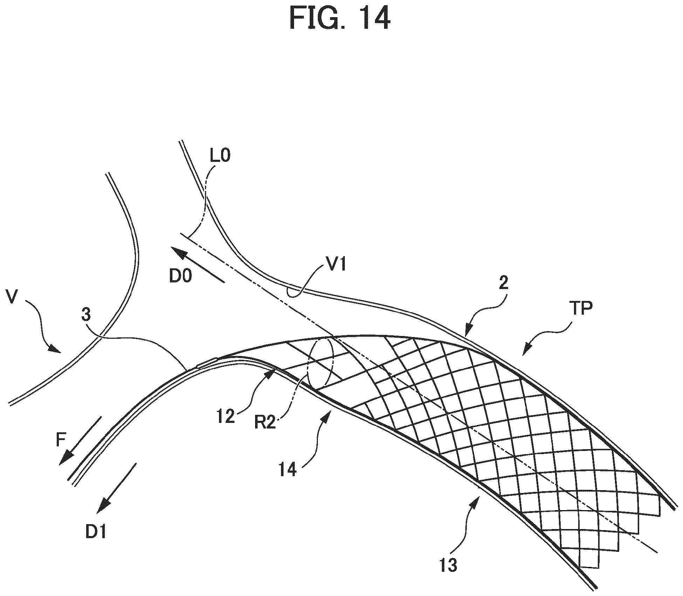

FIG. 13 is an enlarged view showing a shape of the locking stent 2A of the comparative example when a force for pulling the first catheter 5 and the delivery wire 3 toward the proximal side D1 is applied. The locking stent 2A of the comparative example shown in FIG. 13 is a stent of a conventional example configured by cells having the same shape. FIG. 14 is an enlarged view showing the shape of the locking stent 2 of the embodiment when a force is applied to pull the first catheter 5 and the delivery wire 3 to the proximal side D1. In FIGS. 13 and 14, the central axis line L0 of each of the locking stents (2, 2A) indicates an imaginary central axis line in the axial direction of the locking stent locked in the biological lumen V.

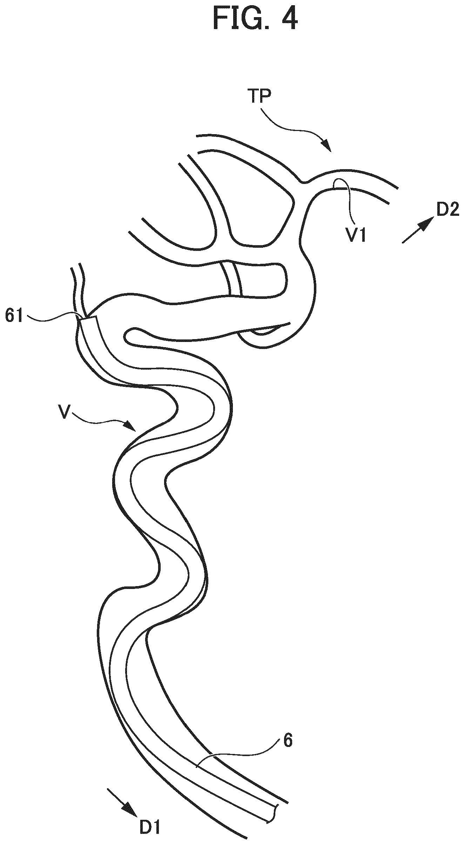

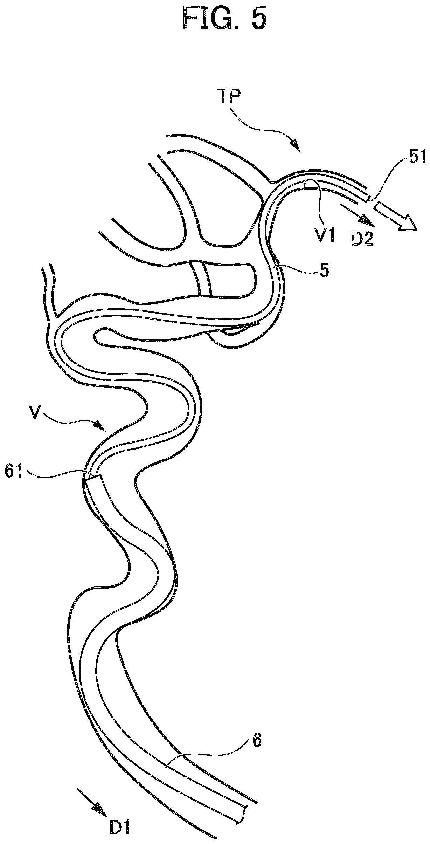

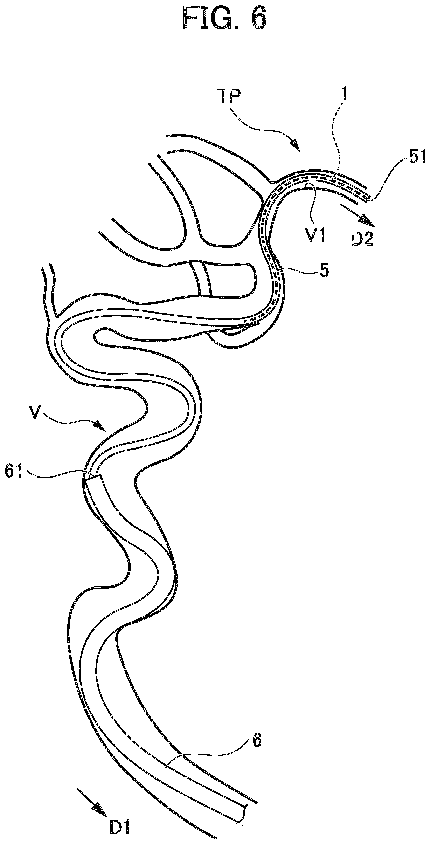

First, the second catheter 6 is disposed on the proximal side D1 of the biological lumen V of a patient. Typically, as shown in FIGS. 3 and 4, a distal end 61 of the second catheter 6 is caught by the bending portion or the branching portion of the biological lumen V, and it is difficult to advance the second catheter 6 further to the distal side. As shown in FIG. 5, the first catheter 5 is inserted into the second catheter 6 to be delivered into the biological lumen V and pushed out of the distal end 61 of the second catheter 6, and then a distal end 51 of the first catheter 5 is disposed in the vicinity of the target position TP. Subsequently, as shown in FIG. 6, the distal stabilizer 1 is inserted into the first catheter 5 and placed in the vicinity of the target position TP. At this time, the locking stent 2 of the distal stabilizer 1 is housed in the first catheter 5 in a state reduced in diameter. In addition, in FIG. 6, the distal stabilizer 1 (the locking stent 2 and the delivery wire 3) is housed over the entire area of the first catheter 5, but for convenience, the distal stabilizer 1 is illustrated by a broken line only on the distal side of the first catheter 5.

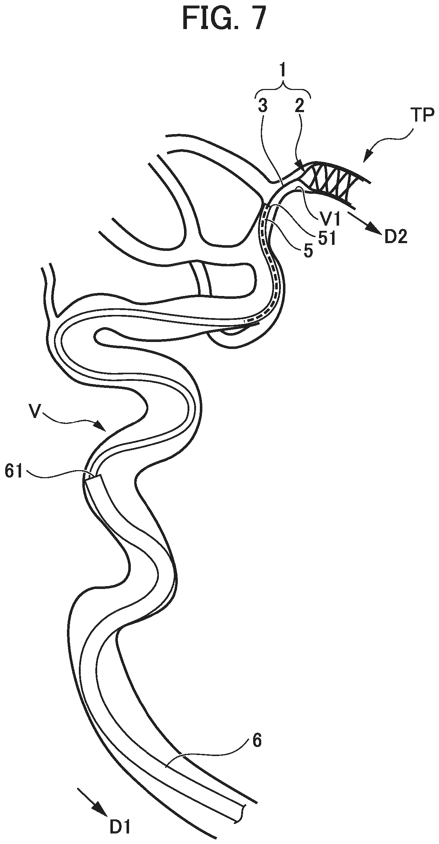

Next, as shown in FIG. 7, the locking stent 2 housed in the first catheter 5 in the state reduced in diameter is released from the distal end 51 of the first catheter 5. Release of the locking stent 2 is performed by an operation of retracting the first catheter 5 toward the proximal side D1. The locking stent 2 released from the distal end 51 of the first catheter 5 expands in diameter due to its self-expanding force. As a result, the locking stent 2 pushes the inner wall V1 of the biological lumen V from the inside toward the outside, and is locked to the inner wall V1.

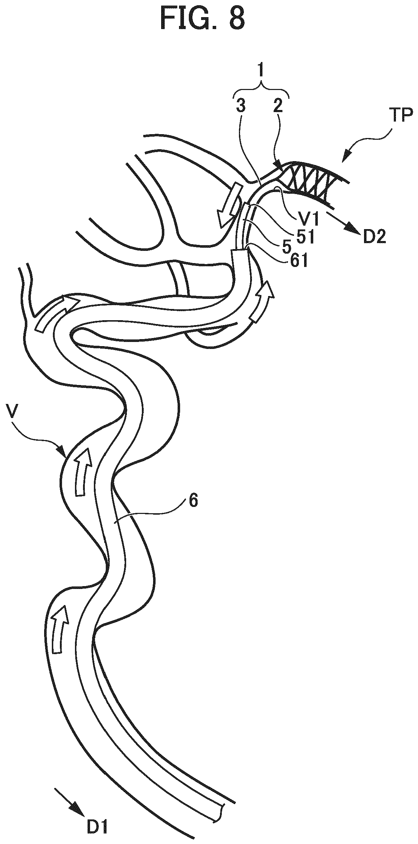

Subsequently, as shown in FIG. 8, the second catheter is slidably fitted over the first catheter 5, and the second catheter 6 is advanced to the distal side D2 in the biological lumen V. The second catheter 6 has a large outer diameter and high rigidity. Therefore, the second catheter 6 repeatedly applies a force to pull the first catheter 5 or the delivery wire 3 having low rigidity toward the proximal side D1 in the process of advancing. As described above, in the locking stent of the conventional example, the position of the locking stent locked to the inner wall in the biological lumen V may be displaced due to the application of the force for pulling the delivery wire toward the proximal side D1.

As shown in FIG. 12, in a state in which the locking stent 2 is locked to the inner wall V1 of the biological lumen V, that is, in a state in which the locking stent 2 is released from the first catheter 5 and deployed (increased in diameter), both the first mesh portion 13 and the second mesh portion 14 support the inner wall V1. Also in the locking stent 2A of the comparative example illustrated in the same drawings, the inner wall V1 is supported over the entire region in the axial direction. In the state shown in FIG. 12, when a force F that pulls the delivery wire 3 toward the proximal side D1 (hereinafter also referred to as "force F") is applied, in the locking stent 2A of the comparative example shown in FIG. 13, the range R1 on the proximal side of the mesh portion 15 on which the force F acts becomes close to the central axis line L0. Hereinafter, a direction D0 is also referred to as a "pulling-out direction D0" or a "direction to pull out D0". As shown in FIG. 13, when the range R1 on the proximal side of the mesh portion 15 is close to the central axis line L0, much of the force F acts in the direction D0 in which the locking stent 2A is pulled out toward the proximal side in the biological lumen V. Therefore, when the delivery wire 3 is pulled toward the proximal side, positional displacement of the locking stent 2A is likely to occur.

On the contrary, in the locking stent 2 of the present embodiment, when the force F for pulling the delivery wire 3 toward the proximal side D1 is applied, as shown in FIG. 14, the second mesh portion 14 having an elongation ratio in the axial direction higher than that of the first mesh portion 13 extends toward the proximal side D1 together with the antenna portion 12, such that the force for pulling the delivery wire 3 toward the proximal side D1 is relaxed by the elongation of the second mesh portion 14, and the force hardly propagates to the first mesh portion 13. Further, in the locking stent 2 of the present embodiment shown in FIG. 14, the range R2 on the proximal side of the second mesh portion 14 on which the force F acts is away from the central axis line L0 of the locking stent 2. As shown in FIG. 14, when the range R2 on the proximal side of the second mesh portion 14 is away from the central axis line L0, the force acting in the direction D0 in which the locking stent 2 is pulled out to the proximal side in the biological lumen V becomes smaller than that in FIG. 13 due to the dispersion of the force F. Therefore, it is possible to more effectively suppress the displacement of the locking stent 2 when the delivery wire 3 is pulled toward the proximal side.

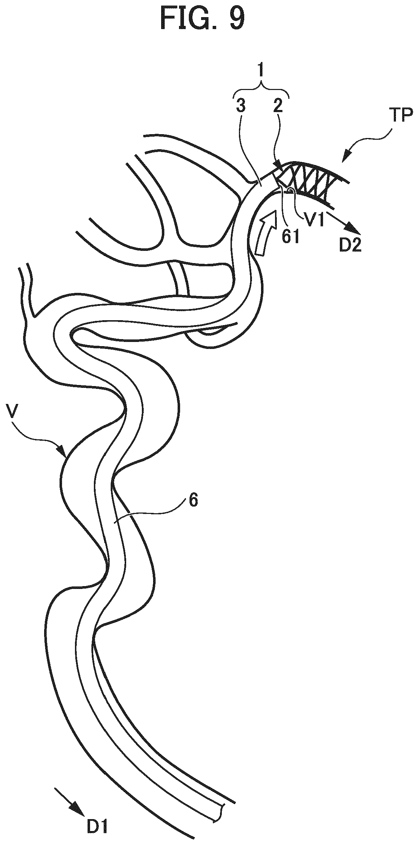

When it is difficult to advance the second catheter 6, an operation of pulling the delivery wire 3 toward the proximal side D1 may be performed in a state where the locking stent 2 is locked to the inner wall V1 of the biological lumen V. By this pulling operation, the second catheter 6 can be advanced toward the distal side D2. Specifically, when the delivery wire 3 is pulled toward the proximal side D1 in a state in which the locking stent 2 is locked to the inner wall V1, the path of the delivery wire 3 in the biological lumen V becomes close to a straight line and becomes short. At this time, when a portion of the proximal side D1 of the second catheter 6 is held directly or indirectly, the position of the portion of the proximal side D1 of the second catheter 6 does not change, and instead, as shown in FIG. 9, the distal end 61 of the second catheter 6 advances in the biological lumen V. Further, when the distal end 61 of the second catheter 6 advances into the biological lumen V without changing the position of the portion of the second catheter 6 on the proximal side D1, the path of the second catheter 6 becomes close to a straight line. This operation is particularly beneficial when the second catheter 6 attempts to pass through a place where it is difficult to pass through, for example, a place where the bending of the biological lumen V is severe or a place where the inner diameter is small. According to the distal stabilizer 1 of the present embodiment, even when such an operation is performed, it is possible to suppress displacement of the locking stent 2 locked to the inner wall V1 of the biological lumen V.

In addition, when a treatment catheter having a large outer diameter is delivered to the vicinity of the aneurysm by locking the locking stent 2 to the distal side of the aneurysm, the first catheter 5 inserted into the treatment catheter (the second catheter 6) in the middle of advancement may stray and enter the aneurysm together with the delivery wire 3. In such a case, by performing an operation of pulling the delivery wire 3 toward the proximal side D1 in a state where the locking stent 2 is locked to the distal side of the aneurysm, the path of the delivery wire 3 in the biological lumen V is shortened, and thus the delivery wire 3 and the first catheter 5 can be returned into the biological lumen V from the aneurysm. As described above, since it is possible for the distal stabilizer 1 of the present embodiment to suppress the displacement of the locking stent 2 locked in the biological lumen V, even when the operation of returning the wire or the catheter which has strayed and entered the aneurysm to the inside of the biological lumen V is performed, it is possible for the distal stabilizer 1 to be suitably used for the application of delivering the treatment catheter to the vicinity of the target position in a state where the locking stent 2 is locked to the distal side of the aneurysm.

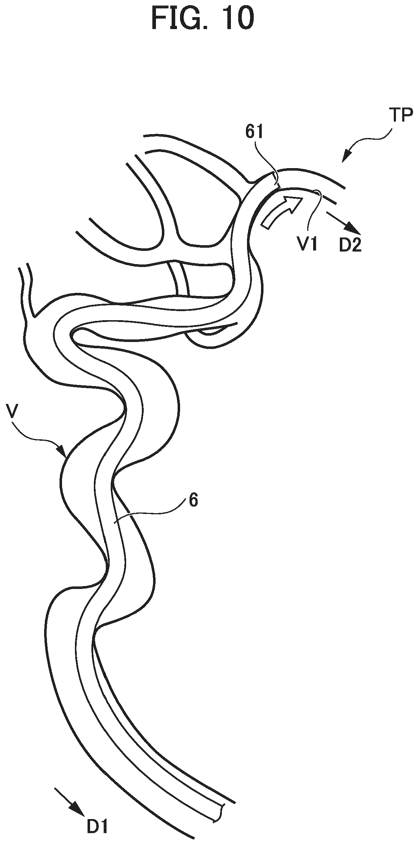

After the delivery of the second catheter 6 is completed in this manner, as shown in FIG. 10, the distal stabilizer 1 is retracted toward the proximal side D1 and housed in the first catheter 5 (not shown). After the distal stabilizer 1 is housed in the first catheter 5, the first catheter 5 and the distal stabilizer 1 may be removed from the proximal side D1 of the second catheter 6. Alternatively, after the first catheter 5 is removed from the proximal side D1, the distal stabilizer 1 may be housed in the second catheter 6 and removed from the proximal side D1. In the distal stabilizer 1 of the present embodiment, since the flexibility of the second mesh portion 14 on the proximal side is high, it is possible for the distal stabilizer 1 to be easily housed (resheathed) in the first catheter 5 or the second catheter 6.

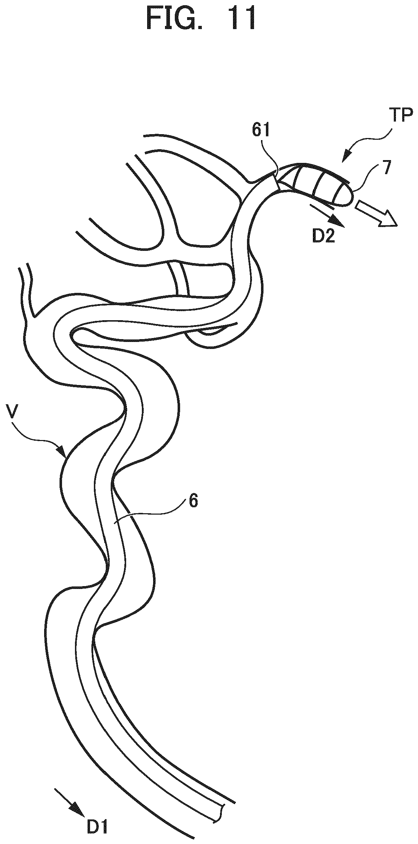

Next, as shown in FIG. 11, by providing the second catheter 6 at the target position TP in the biological lumen V, it is possible to easily deliver a treatment device 7 inserted into the second catheter 6 to the target position TP. Further, the second catheter 6 or the third catheter itself may be used as a treatment device. For example, when the second catheter 6 is used as a thrombus aspiration device, the thrombus at the target position TP is aspirated and removed from the distal end 61 by applying a negative pressure to the internal flow path of the second catheter 6. It should be noted that the removal of the distal stabilizer 1 is not limited to removal before the delivery of the treatment device 7, but may be removal after the delivery of the treatment device 7. In the latter case, the second catheter 6 has at least a double structure of a lumen through which the distal stabilizer 1 and the first catheter 5 are inserted and a lumen through which the treatment device 7 is inserted.

The treatment device 7 is, for example, a thrombus aspiration device, a flow diverter, an aneurysm embolization device, a thrombectomy device (such as a stent retriever), an aneurysm treatment stent, an intracranial arterial stenosis treatment stent, a balloon catheter, a shunt, or a liquid embolic substance release means (such as a catheter with a lumen through which the liquid embolic substance passes). According to the distal stabilizer 1 of the present embodiment, these treatment devices can also be delivered, in terms of transcatheter, to the above-described region of the vessel inner diameter of 0.5 mm to 10 mm, preferably 7 mm or less, specifically less than 2.5 mm (preferably 2.0 mm or less, or 1.5 mm or less), and diseases or conditions (vascular occlusions, aneurysms, etc.) in these regions can be treated or ameliorated with little burden on the patient.

Further, the treatment device 7 delivered to the target position TP may be used in the biological lumen (a thrombus aspiration device, a flow diverter, an aneurysm embolization device, a thrombectomy device, a stent for treating aneurysm, a stent for treating intracranial arterial stenosis, a balloon catheter, or a means for releasing liquid embolic substance, etc.) or may be used by protruding out of the biological lumen (a shunt or the like). The distal stabilizer 1 of the present embodiment is also applied to a method of delivering the treatment device 7 to the target position TP in the biological lumen V by the delivery system 10 for performing a therapeutic action at the target position TP in the biological lumen V, or for causing the treatment device 7 to protrude from the target position TP in the biological lumen V to the outside of the biological lumen V to perform a therapeutic action.

According to the distal stabilizer 1 of the present embodiment, for example, the following advantageous effects are achieved. The distal stabilizer 1 of the embodiment has, on the proximal side of the locking stent 2, the second mesh portion (the easily deformable portion) 14 that is more deformable than the distal side of the locking stent 2. In this configuration, when the delivery wire 3 is pulled toward the proximal side in a state in which the locking stent 2 is locked to the inner wall in the biological lumen, the second mesh portion 14 having an elongation ratio higher than that of the first mesh portion 13 extends to the proximal side, and therefore, the force for pulling the delivery wire 3 to the proximal side is relaxed by the elongation of the second mesh portion 14, and the force hardly propagates to the first mesh portion 13. Therefore, when the delivery wire 3 is pulled toward the proximal side, it is possible to suppress displacement of the locking stent locked to the inner wall in the biological lumen. In addition, in the bent biological lumen V, the entire second mesh portion 14 having low bending rigidity in the axial direction deforms into a substantially conical shape together with the antenna portion 12 and is largely bent in the axial direction along the bending of the biological lumen V. Therefore, in the locking stent 2, the range R2 on the distal side of the second mesh portion 14 on which the force F acts is away from the central axis line L0 of the locking stent 2 (refer to FIG. 14). As described above, when the range R2 on the distal side of the second mesh portion 14 is away from the central axis line L0, the force acting in the direction D0 of pulling out the locking stent 2 to the proximal side in the biological lumen V becomes small due to the dispersion of the force F. Therefore, it is possible to more effectively suppress the displacement of the locking stent 2 when the delivery wire 3 is pulled toward the proximal side.

Since the locking stent 2 of the embodiment is more likely to deform on the proximal side in the axial direction than on the distal side, stress is less likely to concentrate on both end portions in the axial direction of the locking stent 2. According to this configuration, since the blood vessel to be locked is less likely to have a shape close to a straight line, the inner wall of the blood vessel is more protected.

In the locking stent 2 of the embodiment, the second mesh portion 14 has flexibility by making the opening area of each of the cells 17 larger than that of each of the cells 16 of the first mesh portion 13. Therefore, it is possible to set the flexibility (bending rigidity) of the second mesh portion 14 within a desired range by appropriately adjusting the opening area of each of the cells 17 of the second mesh portion 14.

In the locking stent 2 of the embodiment, by setting the ratio of the length of the first mesh portion 13 to the length of the second mesh portion 14 to 1:1 or more, the first mesh portion 13 can easily secure the locking force necessary for locking the locking stent 2 to the inner wall of the biological lumen. Further, by setting the ratio of the length of the first mesh portion 13 to the second mesh portion 14 to be less than 1:4, it is possible to prevent the characteristics (elongation or bending toward the proximal side) of the second mesh portion 14 from being impaired when the delivery wire 3 is pulled toward the proximal side. Therefore, by setting the ratio of the length in the axial direction of the second mesh portion 14 in the main body portion 11 of the locking stent 2 to 1:1 to 1:4, it is possible to sufficiently exhibit the characteristics of the second mesh portion 14 while securing the locking force of the first mesh portion 13.

Although embodiments of the present invention have been described above, the present invention is not limited to the above-described embodiments, and various modifications and changes such as those described below can be made and are also included in the technical scope of the present invention. In addition, the advantageous effects described in the embodiments merely exemplify the most preferable effects generated from the present invention, and are not limited to those described in the embodiments. Although the above-described embodiments and modifications described below can be appropriately combined and used, detailed descriptions thereof will be omitted.

### (Modifications)

As a configuration for making the bending rigidity in the axial direction of the second mesh portion 14 lower than that of the first mesh portion 13, for example, the following configuration may be applied.
(1) A preferable range of the reference opening area may be set as the opening area of each of the cells 17 of the second mesh portion 14, and the opening area of each of the cells 17 of the second mesh portion 14 may be set within the range of the reference opening area. According to this modification, it is possible to prevent the flexibility of the second mesh portion 14 from becoming extremely low.
(2) The cross-sectional area of each of the struts constituting the cells 17 of the second mesh portion 14 may be smaller than the cross-sectional area of each of the struts constituting the cells 16 of the first mesh portion 13. In this modification, the shapes and sizes of the cells of the first mesh portion 13 and the second mesh portion 14 may be the same or different.
(3) The cells 17 of the second mesh portion 14 may be formed with a material having a bending rigidity lower than that of the cells 16 of the first mesh portion 13. The material used for each mesh portion may be selected based on the bending rigidity measured in the bending test under the same conditions. In this modification, the shapes and sizes of the cells of the first mesh portion 13 and the second mesh portion 14 may be the same or different.

### EXPLANATION OF REFERENCE NUMERALS

1 distal stabilizer
2 locking stent
3 delivery wire
10 delivery system
13 first mesh portion
14 second mesh portion (easily deformable portion)
16, 17 cell
TP target position
V biological lumen
V1 inner wall

## Claims

1. A distal stabilizer for use in catheter delivery in a biological lumen, the distal stabilizer comprising:
a linear delivery member; and
a cylindrical portion that extends from a distal end of the linear delivery member and is locked to an inner wall of the biological lumen by a mesh pattern structure,
wherein the cylindrical portion has a range of an effective length, and includes an easily deformable portion on a proximal side of the range of the effective length, such that the proximal side is more deformable than a distal side of the effective length.

2. The distal stabilizer according to claim 1, wherein an opening area of each cell constituting the mesh pattern structure of the easily deformable portion is larger than an opening area of each cell constituting the mesh pattern structure of the distal side of the cylindrical portion.

3. The distal stabilizer according to claim 1, wherein an opening area of each cell constituting the mesh pattern structure of the easily deformable portion is larger than a reference opening area.

4. The distal stabilizer according to any one of claims 1 to 3, wherein a ratio of a length of the easily deformable portion in an axial direction relative to the range of the effective length of the cylindrical portion is 1:0.5 to 1:0.2.
